# EUROPEAN PATENT APPLICATION

(11) **EP 4 205 714 A1**
(43) Date of publication of application: **05.07.2023**
(21) Application number: 21875899.3
(22) Date of filing: 01.10.2021
(51) Int. Cl.: A61F 13/15, A61F 13/49

(54) **WEARABLE ARTICLE MANUFACTURING METHOD AND WEARABLE ARTICLE MANUFACTURED THEREWITH**

(30) Priority: 02.10.2020 JP 2020167749
(71) Applicant: Zuiko Corporation, Ibaraki-shi Osaka 5670082 (JP)
(72) Inventor: SHIMADA, Takahiro, Settsu-shi, Osaka 566-0045 (JP)
(74) Representative: Hasegawa, Kan
(86) International application number: PCT/JP2021/036528
(87) International publication number: WO 2022/071604

(57) **Abstract**

This manufacturing method improves the productivity for wearable articles by efficiently weakening an elastic sheet. This wearable article manufacturing method involves: a sheet preparation step (1-1) in which an elastic sheet (6) for forming a front abdominal part and a back part is prepared; a sheet weakening step (1-2) in which the elasticity of the elastic sheet (6) is weakened in a front weakening region (FW), which is a front connection region (FC) on the elastic sheet (6) for connection of the front part (4a) of an absorbent body (4), and which is pre-set as a region for disposing an absorbent core (4d) of the absorbent body (4), and in a rear weakening region (RW), which is a rear connection region (RC) for connection of the rear part (4b) of the absorbent body (4), and which is pre-set as a region for disposing the absorbent core (4d); and an absorbent body connection step (1-4) in which the front part (4a) of the absorbent body (4) is connected to the front connection region (FC) of the elastic sheet (6), and the rear part (4b) of the absorbent body (4) is connected to the rear connection region (RC) of the elastic sheet (6). The sheet weakening step (1-2) is carried out simultaneously for the front weakening region (FW) and the rear weakening region (RW).

## Description

### Technical Field

The present invention relates to a wearable article.

### Background Art

Conventionally, there has been known a wearable article including a front abdominal part disposed in a wearer's abdomen, a back part disposed in a wearer's buttocks, and an absorbent body having a front part connected to the front abdominal part, a rear part connected to the back part, and an intermediate part extending from the front part to the rear part and disposed in a crotch part of the wearer.

The front abdominal part and the back part have stretchability in a width direction (left-right direction as viewed from the wearer) in order to improve a fit feeling when worn.

For example, an absorbent article described in Patent Literature 1 includes a front panel, a back panel, and an absorbent assembly. The front panel and the back panel have elastic sheets having stretchability.

The elastic sheet has a stretchable first region and a non-stretchable second region. The front part of the absorbent assembly is bonded to the second region of the elastic sheet of the front panel, and the rear part of the absorbent assembly is bonded to the second region of the elastic sheet of the back panel. As a result, expansion and contraction of the absorbent assembly in the width direction is restricted regardless of expansion and contraction of the front panel and the back panel.

Note that Patent Literature 1 describes that elasticity in the second region is invalidated (weakened) by applying heat to the elastic sheet in order to form the second region in the elastic sheet.

However, Patent Literature 1 does not consider a method for weakening an elastic sheet for improving productivity of a wearable article.

### Citation List

### Patent Literature

Patent Literature 1: US Patent No. 7604624

### Summary of Invention

An object of the present invention is to provide a method for manufacturing a wearable article capable of improving the productivity of the wearable article by efficiently weakening the elastic sheet, and the wearable article manufactured by the method.

In order to solve the above problems, the present invention provides a method for manufacturing a wearable article, the wearable article including: a front abdominal part disposed on an abdomen of a wearer; a back part disposed on buttocks of the wearer; and an absorbent body having a front part connected to the front abdominal part, a rear part connected to the back part, and an intermediate part extending from the front part to the rear part and disposed in a crotch part of the wearer. The method includes: a sheet preparation step of preparing at least one elastic sheet for forming the front abdominal part and the back part; a sheet weakening step of weakening elasticity of the elastic sheet in a front weakening region that is a front connection region to which a front part of the absorbent body is connected and that is pre-set as a region where an absorbent core of the absorbent body is disposed and a rear weakening region that is a rear connection region to which a rear part of the absorbent body is connected and that is pre-set as a region where the absorbent core is disposed in the at least one elastic sheet; and an absorbent body connection step of connecting a front part of the absorbent body to a front connection region of the at least one elastic sheet and connecting a rear part of the absorbent body to a rear connection region of the at least one elastic sheet. The sheet weakening step is simultaneously performed for the front weakening region and the rear weakening region.

Further, the present invention provides a wearable article manufactured by the method for manufacturing a wearable article.

According to the present invention, it is possible to improve the productivity of the wearable article by efficiently weakening the elastic sheet.

### Brief Description of Drawings

FIG. 1 is a process diagram illustrating a method for manufacturing a wearable article according to a first embodiment of the present invention.
FIG. 2 is a front view illustrating a schematic configuration of a weakening treatment device for executing a sheet weakening step in FIG. 1.
FIG. 3 is a plan view of another wearable article that can be manufactured by changing a part of the method for manufacturing the wearable article illustrated in FIG. 1.
FIG. 4 is a process diagram illustrating a method for manufacturing a wearable article according to a second embodiment of the present invention.
FIG. 5 is a process diagram illustrating a method for manufacturing a wearable article according to a third embodiment of the present invention.
FIG. 6 is a process diagram illustrating a method for manufacturing a wearable article according to a fourth embodiment of the present invention.
FIG. 7 is a process diagram illustrating a method for manufacturing a wearable article according to a fifth embodiment of the present invention.
FIG. 8 is a process diagram illustrating a method for manufacturing a wearable article according to a sixth embodiment of the present invention.
FIG. 9 is a process diagram illustrating a method for manufacturing a wearable article according to a seventh embodiment of the present invention.
FIG. 10 is a cross-sectional view taken along line X-X of FIG. 9.

### Description of Embodiments

Hereinafter, embodiments of the present invention will be described with reference to the accompanying drawings. Note that the following embodiments are examples embodying the present invention, and are not intended to limit the technical scope of the present invention.

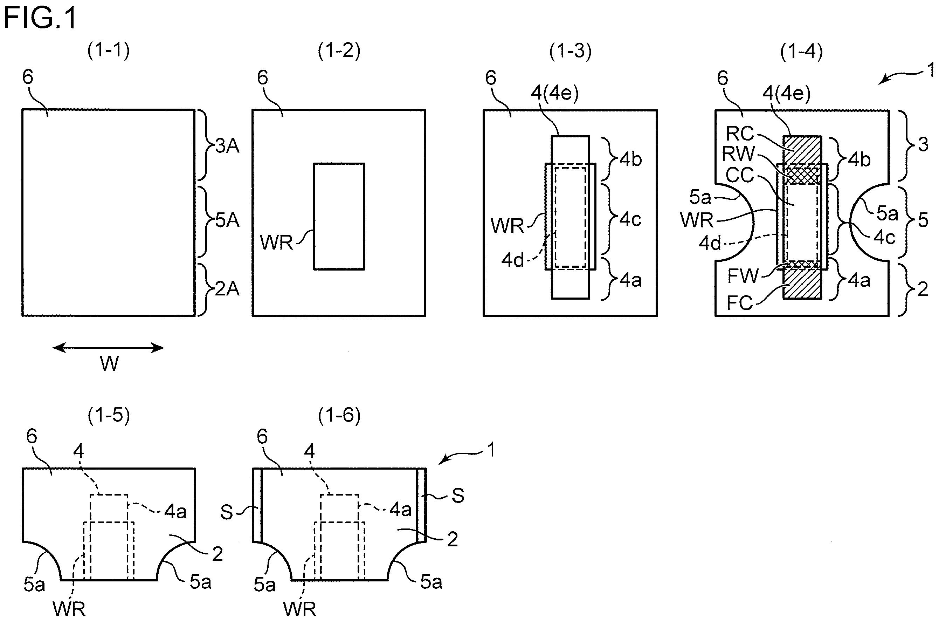

### <First Embodiment (FIGS. 1 to 3)>

FIG. 1 is a process diagram of a method for manufacturing a wearable article (for example, diapers and sanitary shorts) according to a first embodiment of the present invention.

First, a configuration of a wearable article 1 to be manufactured will be described with reference to a step (1-4) and a step (1-6) in FIG. 1.

First, referring to the step (1-4) indicating the unfolded state of the wearable article 1, the wearable article 1 includes an elastic sheet 6 having elasticity in a width direction W (see the step (1-1)) and an absorbent body 4 provided on the elastic sheet 6.

The elastic sheet 6 includes a front elastic portion 2 for forming a front abdominal part disposed on an abdomen of a wearer, a rear elastic portion 3 for forming a back part disposed on buttocks of the wearer, and a crotch part 5 provided between the front elastic portion 2 and the rear elastic portion 3. Leg cutouts 5a for forming leg holes are formed on both sides of the absorbent body 4 in the crotch part 5.

The elastic sheet 6 includes a sheet-like elastic member body (not illustrated) formed of an elastic thermoplastic resin, and a pair of nonwoven fabric sheets (not illustrated) ultrasonically welded to the elastic member body in a state of interposing the elastic member body. The elastic thermoplastic resin is preferably one having elasticity at room temperature, and can be appropriately selected from, for example, thermoplastic elastomers classified in JIS K 6418:2007 (ISO 18064:2003). For example, a thermoplastic elastomer in which the glass transition temperature of the hard segment is about 100°C to 200°C and the glass transition temperature of the soft segment is -70°C to -10°C can be used. Further, the elastic sheet 6 can be manufactured using, for example, the device and method described in WO 2019/155765.

The absorbent body 4 includes a front part 4a connected to the front elastic portion 2, a rear part 4b connected to the rear elastic portion 3, and an intermediate part 4c extending from the front part 4a to the rear part 4b and disposed in the crotch part of the wearer. Hereinafter, a direction in which the absorbent body extends is referred to as an extending direction, and the width direction W is a direction orthogonal to the extending direction (corresponding to the orthogonal direction).

Specifically, the front part 4a of the absorbent body 4 is connected to a front connection region FC (hatched region in FIG. 1) set in the middle in the width direction W of the front elastic portion 2 of the elastic sheet 6. The rear part 4b of the absorbent body is connected to a rear connection region RC (hatched region in FIG. 1) set in the middle in the width direction W in the rear elastic portion 3 of the elastic sheet 6. The intermediate part 4c of the absorbent body is connected to an intermediate connection region CC (a region between the connection regions FC and RC) set in the middle in the width direction W of the crotch part 5 of the elastic sheet 6. These connections can be realized by adhesion using an adhesive or welding using heat or ultrasonic waves.

The absorbent body 4 includes an absorbent core 4d for absorbing urine or the like of a wearer, and a pair of cover sheets 4e connected to the absorbent core 4d in a state where the absorbent core 4d is interposed between front and back surfaces. The pair of cover sheets 4e includes a front surface sheet (reference numeral is omitted) disposed on the front side (the skin surface side of the wearer) of the absorbent core 4d and formed of a material through which liquid can pass, and a back surface sheet (reference numeral is omitted) disposed on the back side (the opposite side to the skin surface) of the absorbent core 4d and formed of a material through which liquid cannot pass.

The absorbent body 4 is connected to the elastic sheet 6 in a state of suppressing application of elastic force by the elastic sheet 6 to the absorbent core 4d. Specifically, in the elastic sheet 6, a region to which the absorbent body is to be connected and in which the absorbent core 4d is to be disposed is subjected to weakening treatment for weakening the elasticity of the elastic sheet 6. In the present embodiment, the weakening region WR including the entire connection region of the absorbent core 4d in the elastic sheet 6 in plan view is subjected to the weakening treatment. That is, the weakening region WR is a region including the entirety of a front weakening region FW (cross-hatched region) pre-set as the region in which the absorbent core 4d is to be disposed in the front connection region FC, a rear weakening region RW (cross-hatched region) pre-set as the region in which the absorbent core 4d is to be disposed in the rear connection region RC, and an intermediate weakening region (reference numeral is omitted: a region between both the regions FW and RW in the absorbent core 4d) pre-set as the region in which the absorbent core 4d is to be disposed in the intermediate connection region CC. In the present embodiment, the weakening region WR is set as a region larger than the absorbent core 4d in the extending direction and the width direction W in plan view. As a result, it is possible to prevent the elastic sheet 6 from imparting elasticity to the absorbent core 4d, so that urine and the like can be reliably absorbed by suppressing deformation of the absorbent core 4d at the time of wearing.

With reference to the step (1-6) indicating the completed state of the wearable article 1 in addition to the step (1-4), both edges in the width direction of the front elastic portion 2 and the rear elastic portion 3 are connected to each other by a side seal part S in a state where the elastic sheet 6 is folded in two such that the front elastic portion 2 and the rear elastic portion 3 overlap each other. The side seal part S can be formed by adhesion using an adhesive or welding using heat or ultrasonic waves. As described above, the wearable article 1 in which the front elastic portion 2 and the rear elastic portion 3 are connected to each other by the side seal part can be worn by passing both legs through leg holes like pants.

Hereinafter, a method for manufacturing the wearable article 1 will be described with reference to FIG. 1.

The method for manufacturing the wearable article 1 includes a sheet preparation step (1-1), a sheet weakening step (1-2), an absorbent body connection step (1-3), a cutout forming step (1-4), a folding-in-half step (1-5), and a side sealing step (1-6).

In the sheet preparation step (1-1), the elastic sheet 6 for forming the front elastic portion 2 and the rear elastic portion 3 is prepared. In the sheet preparation step (1-1) of the present embodiment, one elastic sheet 6 including a front elastic portion 2A for forming the front elastic portion 2, a rear elastic portion 3A for forming the rear elastic portion 3, and a crotch elastic portion 5A for forming the crotch part 5 is prepared.

The steps after the sheet preparation step (1-1) are performed in a state where the elastic sheet 6 is conveyed to one side in the width direction W in a state where tension is applied in the width direction W.

In the sheet weakening step (1-2), as shown in the step (1-4), the elasticity in the weakening region WR including the front weakening region FW, the rear weakening region RW, and the intermediate weakening region of the elastic sheet 6 is weakened. The sheet weakening step (1-2) is executed for the one elastic sheet 6, and is simultaneously executed for the front weakening region FW, the rear weakening region RW, and the intermediate weakening region.

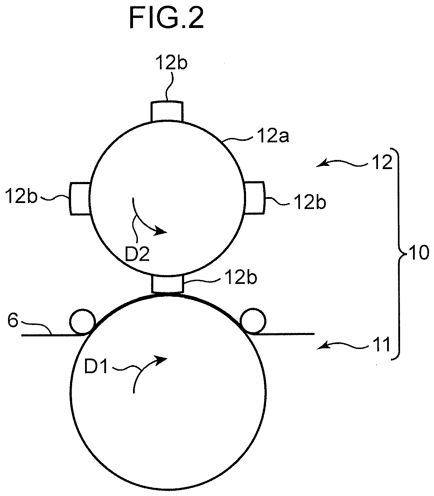

FIG. 2 is a front view illustrating a schematic configuration of a weakening treatment device 10 for executing a weakening step.

Referring to FIG. 2, the weakening treatment device 10 includes a support roller 11 that supports the elastic sheet 6, and a heat roller 12 that applies heat to the elastic sheet 6 supported by the support roller 11. The support roller 11 is a roller that rotates in a direction D1. The heat roller 12 includes a roller main body 12a that rotates in a direction D2 opposite to the direction D1, and a press projection 12b that protrudes outward in the radial direction from a plurality of positions different in the circumferential direction on the outer peripheral surface of the roller main body 12a. The press projection 12b is heated by a heater (not illustrated) and has an outer peripheral surface having an area equivalent to the area of the weakening region WR. When both the rollers 11 and 12 rotate and the elastic sheet 6 is supplied between the press projection 12b and the support roller 11, a portion of the elastic sheet 6 which is pressed and heated between the support roller 11 and the press projection 12b is deformed. As a result, the elasticity in the weakening region WR of the elastic sheet 6 becomes lower than that in a region other than the weakening region WR of the elastic sheet 6, or is invalidated.

In the absorbent body connection step (1-3), the absorbent body 4 is connected to the elastic sheet 6 in a state where the absorbent core 4d is positioned on the elastic sheet 6 so that the absorbent core 4d fits within the weakening region WR. Specifically, the front part 4a of the absorbent body 4 is connected to the front connection region FC, the rear part 4b of the absorbent body 4 is connected to the rear connection region RC, and the intermediate part 4c of the absorbent body 4 is connected to the intermediate connection region CC.

In the present embodiment, the intermediate part 4c of the absorbent body 4 is connected to the elastic sheet 6, but this connection may be omitted.

In the cutout forming step (1-4), the leg cutout 5a is formed along a hole forming region pre-set as a region for forming a leg hole for passing the legs of the wearer. As a result, the elastic sheet 6 is divided into the front elastic portion 2, the rear elastic portion 3, and the crotch part 5.

In the folding-in-half step (1-5), the elastic sheet 6 is folded in half so that the front elastic portion 2 and the rear elastic portion 3 overlap each other.

In the side sealing step (1-6), the ends in the width direction W of the front elastic portion 2 and the rear elastic portion 3 in the elastic sheet 6 folded in half are connected to each other. Thus, the pants-type wearable article 1 having the leg holes formed by the leg cutouts 5a is completed.

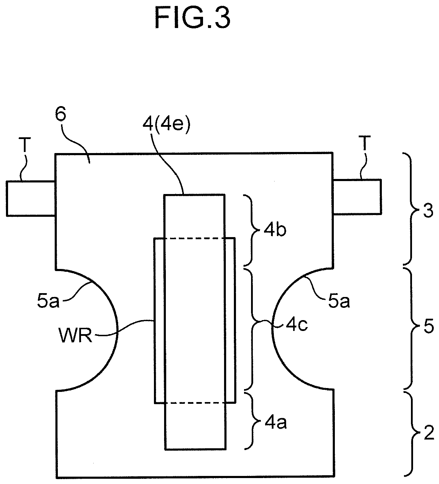

In the above embodiment, the pants-type wearable article 1 is manufactured by performing the side sealing step (1-6), but a tape-type wearable article 1 as illustrated in FIG. 3 can be manufactured by adopting a step of attaching a surface tape T to the elastic sheet 6 instead of the side sealing step (1-6).

The tape-type wearable article 1 includes the surface tape T that can be detachably locked to a surface of the front elastic portion 2 opposite to the skin surface. The surface tape T can be formed by, for example, one surface fastener including a male fastener having a hook part and a female fastener having a loop part. In this case, a male fastener or a female fastener that can be locked to the surface tape T is attached to the front elastic portion 2. A nonwoven fabric can be substituted for the loop part.

In the case of manufacturing the tape-type wearable article 1, a step of attaching the surface tape T and a member (for example, one surface fastener) to be locked thereto to the elastic sheet 6 is performed between steps (1-1) to (1-5) illustrated in FIG. 1.

In the above embodiment, the method for manufacturing the wearable article 1 using the elastic sheet 6 cut in the size in the width direction W of the wearable article 1 has been described, but it is preferable to manufacture the wearable article 1 using a continuous body in which a plurality of elastic sheets 6 are continuous in the width direction W. In the case of manufacturing the pants-type wearable article illustrated in FIG. 1, the continuous body can be cut into individual elastic sheets 6 after the side sealing step (1-6). When the tape-type wearable article 1 illustrated in FIG. 3 is manufactured, the continuous body can be cut into individual elastic sheets 6 before the step of attaching the surface tape T.

As described above, the front connection region FC and the rear connection region RC in the elastic sheet 6 for forming the front elastic portion 2 and the rear elastic portion 3 can be simultaneously weakened in elasticity. As a result, the efficiency of the weakening step (1-2) can be improved as compared with the case where the front connection region FC and the rear connection region RC are individually weakened in elasticity. Therefore, the productivity of the wearable article 1 can be improved.

Further, according to the first embodiment, by performing the weakening step on one elastic sheet 6, the elasticity in the front connection region FC and the rear connection region RC can be simultaneously weakened. Therefore, the productivity of the wearable article can be improved as compared with the case of executing the weakening step while adjusting the relative positions of the two elastic sheets 6.

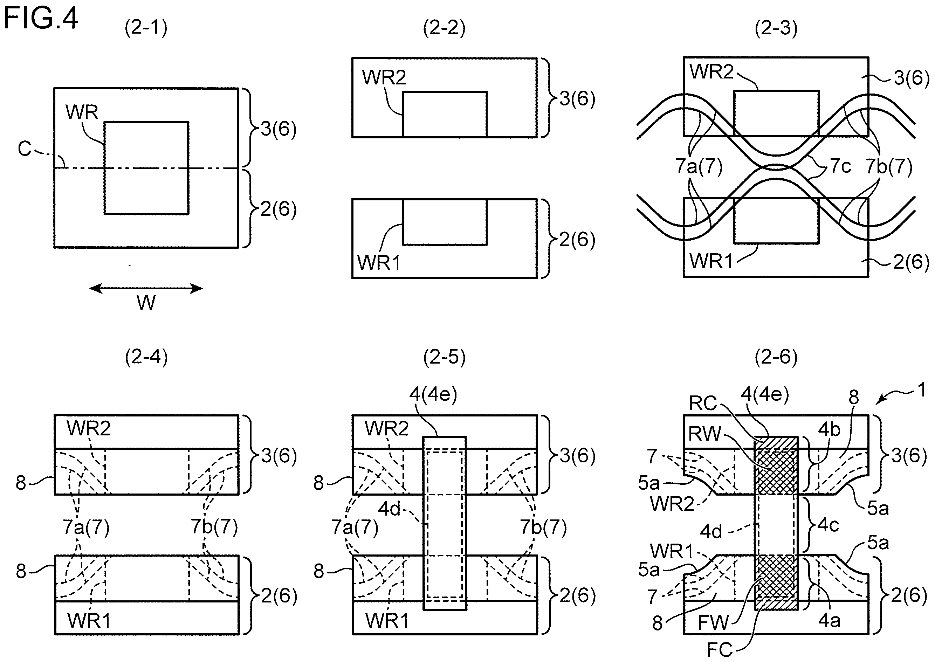

### <Second Embodiment (FIG. 4)>

In the first embodiment, the elastic sheet 6 includes the crotch part 5, but the crotch part 5 may be omitted as illustrated in the second embodiment illustrated in FIG. 4. Unlike the first embodiment, the wearable article 1 according to the second embodiment includes a leg elastic member 7 and a covering sheet 8 covering the leg elastic member 7.

Hereinafter, a configuration and a manufacturing method of the wearable article 1 according to the second embodiment will be described. In the second embodiment, the same parts as those in the first embodiment are denoted by the same reference numerals, and the description thereof will be omitted.

Referring to the step (2-6) of FIG. 4, the wearable article 1 includes the front elastic portion 2 and the rear elastic portion 3 having elasticity in the width direction W, and the absorbent body 4 disposed so as to extend across the elastic portions 2 and 3 and connected to the elastic portions 2 and 3.

The front elastic portion 2 is a portion for forming the front abdominal part to be disposed in the abdomen of the wearer. The leg cutouts 5a for forming leg holes are formed at both ends of the front elastic portion 2 in the width direction W.

The rear elastic portion 3 is a portion for forming a back part to be disposed on the buttocks of the wearer. The leg cutouts 5a for forming leg holes are formed at both ends of the rear elastic portion 3 in the width direction W.

The absorbent body 4 includes the front part 4a connected to the front connection region FC (hatched region) of the front elastic portion 2, the rear part 4b connected to the rear connection region RC (hatched region) of the rear elastic portion 3, and the intermediate part 4c disposed between the front elastic portion 2 and the rear elastic portion 3.

The absorbent body 4 is connected to both the elastic portions 2 and 3 in a state where the absorbent core 4d is prevented from receiving elastic force from both the elastic portions 2 and 3 in a state of being connected to both the elastic portions 2 and 3. Specifically, a weakening region WR1 in which the elasticity of the front elastic portion 2 is weakened is formed in the front elastic portion 2, and a weakening region WR2 in which the elasticity of the rear elastic portion 3 is weakened is formed in the rear elastic portion 3. The weakening region WR1 is a region including the front weakening region FW pre-set as a region in which the absorbent core 4d is to be disposed in the front connection region FC. The weakening region WR2 is a region including the rear weakening region RW pre-set as a region in which the absorbent core 4d is to be disposed in the rear connection region RC.

The leg elastic members 7 are provided at elastic member connection positions pre-set on both sides of the region where the absorbent core 4d is to be disposed in the width direction W. Specifically, the leg elastic member 7 is a yarn or string-like elastic member, and is connected in an extended state along the extending direction (along the leg cutout 5a) with respect to the surfaces of the elastic portions 2 and 3 facing the skin of the wearer. In the present embodiment, two leg elastic members 7 are provided on both sides of the absorbent core 4d of the front elastic portion 2, and two leg elastic members 7 are provided on both sides of the absorbent core 4d of the rear elastic portion 3. A plurality of leg elastic members 7 (two in the example of FIG. 4) are provided in the vicinity of each leg cutout 5a.

The covering sheet 8 is provided on a surface of each of the front elastic portion 2 and the rear elastic portion 3 facing the skin of the wearer so as to cover the leg elastic member 7. That is, in a state where the leg elastic member 7 is interposed between the front elastic portion 2 and the covering sheet 8, the front elastic portion 2 and the covering sheet 8 are connected, and at least one of the front elastic portion 2 and the covering sheet 8 is connected to the leg elastic member 7. Similarly, in a state where the leg elastic member 7 is interposed between the rear elastic portion 3 and the covering sheet 8, the rear elastic portion 3 and the covering sheet 8 are connected, and at least one of the rear elastic portion 3 and the covering sheet 8 is connected to the leg elastic member 7. These connections can be realized by adhesion using an adhesive or welding using heat or ultrasonic waves. The covering sheet 8 is a sheet formed of a nonwoven fabric.

Hereinafter, a method for manufacturing the wearable article 1 will be described with reference to FIG. 4. Description of steps similar to those in the method for manufacturing the wearable article 1 of the first embodiment will be omitted.

The method for manufacturing the wearable article 1 includes a sheet preparation step (not illustrated), a sheet weakening step (2-1), a cutting and separating step (2-2), an elastic member supply step (2-3), a sheet connection step (2-4), an absorbent body connection step (2-5), and a cutout forming step (2-6). In addition, the method for manufacturing the wearable article 1 may include the folding-in-half step (1-5) in the first embodiment in addition to the above steps, and may include the side sealing step (1-6) or the step of attaching the surface tape T illustrated in FIG. 3. Through the above steps, the pants-type wearable article 1 (see FIG. 1) or the tape-type wearable article 1 (see FIG. 3) can be manufactured.

In the sheet preparation step, as shown in the sheet weakening step (2-1), one elastic sheet 6 having the front elastic portion 2 and the rear elastic portion 3 is prepared.

In addition, the steps after the sheet preparation step are executed in a state where the elastic sheet 6 is conveyed to one side in the width direction W in a state where tension is applied in the width direction W.

In the sheet weakening step (2-1), the elasticity in the weakening region WR including the weakening region WR1 and the weakening region WR2 of the elastic sheet 6 is weakened. The sheet weakening step (2-1) is executed for one elastic sheet 6, and is simultaneously executed for the weakening regions WR1 and WR2.

In the cutting and separating step (2-2), after the sheet weakening step (2-1), one elastic sheet 6 is cut into the front elastic portion 2 and the rear elastic portion 3 along a pre-set cutting line C. As a result, the weakening region WR is divided into the weakening region WR1 and the weakening region WR2. Further, in the cutting and separating step (2-2), the cut front elastic portion 2 and the cut rear elastic portion 3 are separated from each other in the extending direction.

In the elastic member supply step (2-3), the leg elastic members 7 are respectively disposed at elastic member connection positions (positions indicated by broken lines in step (2-4)) pre-set on both sides of the region where the absorbent core 4d is to be disposed in the width direction W, and the leg elastic members 7 are supplied to the front elastic portion 2 and the rear elastic portion 3 in a state where the leg elastic members 7 extend in the extending direction.

Specifically, in the elastic member supply step (2-3), the leg elastic member 7 is supplied along a path (a path along a sine wave in the illustrated example) meandering with respect to the conveyance direction (one side in the width direction W) of the elastic sheet 6 (the front elastic portion 2 and the rear elastic portion 3). That is, in the elastic member supply step (2-3), the leg elastic member 7 is connected to the front elastic portion 2 and the rear elastic portion 3 so that a first portion 7a, a second portion 7b, and a connection portion 7c are formed. The first portion 7a is connected in an extended state in the extending direction with respect to the elastic member connection position set on the left side of the region where the absorbent core 4d is to be disposed. The second portion 7b is connected in an extended state in the extending direction with respect to the elastic member connection position set on the right side of the region where the absorbent core 4d is to be disposed. The connection portion 7c connects the first portion 7a and the second portion 7b, and is disposed outside the front elastic portion 2 and the rear elastic portion 3.

Next, in the sheet connection step (2-4), the covering sheet 8 is disposed for each of the front elastic portion 2 and the rear elastic portion 3 so as to cover the first portion 7a and the second portion 7b of the leg elastic member 7, and the covering sheet 8 is connected to the front elastic portion 2 and the rear elastic portion 3. In the sheet connection step (2-4), in the present embodiment, the covering sheet 8 is disposed with respect to the front elastic portion 2 and the rear elastic portion 3 so as to cover the weakening regions WR1 and WR2, respectively.

Specifically, in the sheet connection step (2-4), an adhesive is supplied to the covering sheet 8, and the covering sheet 8 and the front elastic portion 2 and the covering sheet 8 and the rear elastic portion 3 are pressed with nip rolls (not illustrated) in a state where the covering sheet 8 to which the adhesive is applied overlaps the front elastic portion 2 and the rear elastic portion 3. Thus, the covering sheet 8 is bonded to each of the front elastic portion 2 and the rear elastic portion 3.

In the present embodiment, the leg elastic member 7 is bonded to each of the front elastic portion 2 and the rear elastic portion 3 with an adhesive in a state where the first portion 7a and the second portion 7b of the leg elastic member 7 are extended in the extending direction by the interposing pressure using the nip roll (not illustrated) in the sheet connection step (2-4). That is, the elastic member supply step (2-3) and the sheet connection step (2-4) correspond to the elastic member connection step in the present embodiment. The supply of an adhesive may be performed before or after the supply of the leg elastic member 7 in the elastic member supply step (2-3), and may be performed on the elastic sheet 6 (the front elastic portion 2 and the rear elastic portion 3) instead of the covering sheet 8.

Although the connection of the leg elastic member 7 using an adhesive has been described as an example, in addition to or instead of the adhesive, the front elastic portion 2 and the rear elastic portion 3 can be connected to the covering sheet 8, and the front elastic portion 2 and the rear elastic portion 3 can be connected to the leg elastic member 7 by welding with heat or ultrasonic waves. In this case, in the elastic member supply step (2-3), the leg elastic member 7 can be ultrasonically welded to the front elastic portion 2 and the rear elastic portion 3.

After the leg elastic member 7 is connected to the front elastic portion 2 and the rear elastic portion 3 as described above, the portion of the leg elastic member 7 disposed outside the front elastic portion 2 and the rear elastic portion 3, that is, the connection portion 7c is separated from the first portion 7a and the second portion 7b (elastic member weakening step). As a result, the elasticity of the region pre-set as the region where the absorbent core 4d is to be disposed in the connection portion 7c is weakened (invalidated in the present embodiment).

In the absorbent body connection step (2-5), the front part 4a of the absorbent body 4 is connected to the front connection region FC (hatched portion in step (2-6)), and the rear part 4b of the absorbent body 4 is connected to the rear connection region RC (hatched portion in step (2-6)).

In the cutout forming step (2-6), the leg cutout 5a along the pre-set hole forming region is formed in the front elastic portion 2 and the rear elastic portion 3.

According to the second embodiment, by connecting the absorbent body 4 to two elastic sheets (the front elastic portion 2 and the rear elastic portion 3) so as to extend across the two elastic sheets separated from each other, a region between the elastic portions 2 and 3 and divided by the absorbent body 4 can be used as a leg hole. Therefore, the elastic sheet in the portion covering the crotch part of the wearer can be omitted, and the usage amount of the elastic sheet 6 can be reduced.

Further, by connecting the leg elastic member 7 to the elastic sheet 6 (the front elastic portion 2 and the rear elastic portion 3) in a state of extending in the extending direction, elasticity in the extending direction can be imparted to the elastic sheet 6. Therefore, it is not necessary to extend the elastic sheet 6 in a plurality of directions (the width direction W and the extending direction) in order to impart elasticity of the elastic sheet 6 itself in the extending direction, and handling of the elastic sheet 6 becomes easy.

In addition, by interposing the leg elastic member 7 between the covering sheet 8 and the elastic sheet 6, it is possible to sufficiently secure a holding force for holding the leg elastic member 7 with respect to the elastic sheet 6. Therefore, the leg elastic member 7 can be reliably connected to the elastic sheet 6.

Further, as described above, after the leg elastic member 7 is connected to the front elastic portion 2 and the rear elastic portion 3, the elasticity of the region where the absorbent core 4d is disposed in the connection portion 7c of the leg elastic member 7 is weakened (invalidated). As a result, it is possible to prevent elasticity by the connection portion 7c from being imparted to the absorbent core.

Note that the elastic member supply step (2-3) of the second embodiment can also be applied to a case where the elastic sheet 6 has a portion that covers the region of the crotch part of the wearer as in the first embodiment. In this case, the same effect as described above can be obtained by weakening the elasticity of the region where the absorbent core 4d is to be disposed in the connection portion 7c. Further, the sheet connection step (2-4) can also be applied to the first embodiment.

Further, in the second embodiment, it has been described that the steps (2-1) to (2-6) are performed using the individual elastic sheets 6, but it is preferable to perform the steps (2-1) to (2-6) in a state where the continuous body in which the plurality of elastic sheets 6 is continuous in the width direction W is conveyed in the width direction W.

Further, although an example in which the absorbent body connection step (2-5) is performed before the cutout forming step (2-6) has been described, the order thereof may be reversed.

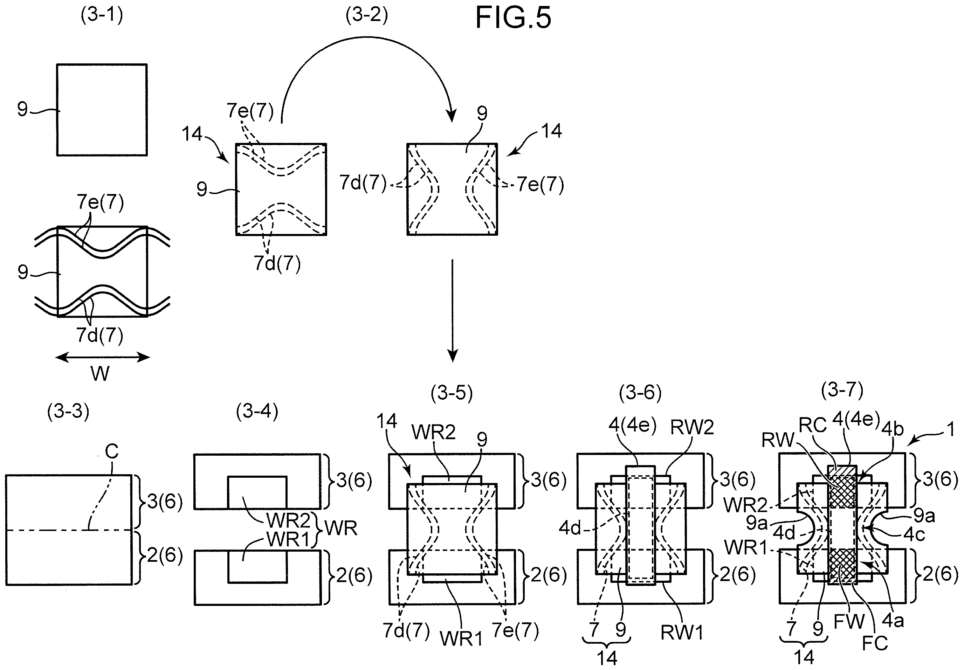

### <Third Embodiment (FIG. 5)>

In the second embodiment, an example in which the leg elastic member 7 is directly connected to the elastic sheet 6 has been described. On the other hand, as in the third embodiment illustrated in FIG. 5, an elastic component 14 having the leg elastic member 7 and a pair of interposing sheets (an example of a holding sheet) 9 interposing the leg elastic member 7 may be connected to the elastic sheet 6. In addition, in the third embodiment, by disposing the elastic component 14 so as to extend across the front elastic portion 2 and the rear elastic portion 3, the leg elastic member 7 can also be provided in a region disposed in the crotch part of the wearer.

Hereinafter, a configuration and a method for manufacturing the wearable article 1 according to the third embodiment will be described. In the third embodiment, the same components as those of the second embodiment are denoted by the same reference numerals, and the description thereof will be omitted.

Referring to the step (3-7) of FIG. 5, the wearable article 1 includes the front elastic portion 2 and the rear elastic portion 3 having elasticity in the width direction W, the elastic component 14 disposed so as to extend across the elastic portions 2 and 3 and connected to the elastic portions 2 and 3, and the absorbent body 4 disposed so as to overlap the elastic component 14 and extend across the elastic portions 2 and 3 and connected to the elastic component 14 and the elastic portions 2 and 3.

The elastic component 14 is connected to the front elastic portion 2 and the rear elastic portion 3 by bonding with an adhesive or welding with heat or ultrasonic waves. In addition, the elastic component 14 includes the leg elastic members 7 provided at elastic member connection positions pre-set on both sides of the region where the absorbent core 4d is to be disposed in the width direction W, and a pair of interposing sheets 9 interposing the leg elastic members 7.

The interposing sheet 9 includes a front part disposed so as to overlap the front elastic portion 2, a rear part disposed so as to overlap the rear elastic portion 3, and an intermediate part between the front portion and the rear portion, and is formed of a nonwoven fabric. A leg cutout 9a for forming a leg hole is formed in the intermediate part of the interposing sheet 9.

The leg elastic members 7 are provided at elastic member connection positions pre-set on both sides in the width direction W of the region where the absorbent core 4d is to be disposed in the interposing sheet 9. Specifically, the leg elastic members 7 are connected in an extended state in the extending direction so as to avoid the leg cutout 9a over the region from the front part to the rear part of the interposing sheet 9.

Hereinafter, a method for manufacturing the wearable article 1 will be described with reference to FIG. 5. Description of steps similar to those in the method for manufacturing the wearable article 1 of the second embodiment will be omitted.

The method for manufacturing the wearable article 1 includes an elastic component preparation step (3-1), a posture changing step (3-2), a sheet preparation step (3-3), a sheet weakening step (not illustrated), a cutting and separating step (3-4), an elastic member connection step (3-5), an absorbent body connection step (3-6), and a cutout forming step (3-7). In addition, the method for manufacturing the wearable article 1 may include the folding-in-half step (1-5) in the first embodiment in addition to the above steps, and may include the side sealing step (1-6) or the step of attaching the surface tape T illustrated in FIG. 3. Through the above steps, the pants-type wearable article 1 (see FIG. 1) or the tape-type wearable article 1 (see FIG. 3) can be manufactured.

In the elastic component preparation step (3-1), one interposing sheet (hereinafter, referred to as a first interposing sheet 9) 9 is conveyed in the width direction W, and a pair of leg elastic members 7d and 7e (two leg elastic members 7d and two leg elastic members 7e in the present embodiment) is supplied in a state of being extended in the width direction W with respect to the first interposing sheet 9 and being separated from each other in the extending direction. Specifically, the leg elastic members 7d and 7e are supplied along a path (a path along a sine wave in the illustrated example) meandering in the conveyance direction (width direction W) of the first interposing sheet 9. Here, the path for supplying the leg elastic members 7d and 7e is set so as to avoid the formation position of the leg cutout 9a set at the central position in the width direction W of the first interposing sheet 9.

Further, in the elastic component preparation step (3-1), an adhesive is applied to the other interposing sheet 9 (hereinafter, referred to as a second interposing sheet 9), the second interposing sheet 9 is supplied to the first interposing sheet 9 so as to interpose the adhesive, and both the interposing sheets 9 are pressed by nip rolls (not illustrated) in a state where the interposing sheets 9 are overlapped. As a result, the leg elastic members 7d and 7e are connected to the interposing sheet 9, and the interposing sheets 9 are joined to each other. The connection is not limited to connection by an adhesive, and may be connection by welding using heat or ultrasonic waves. Further, one of the two interposing sheets 9 may be omitted, and the leg elastic members 7d and 7e may be connected (held) to one interposing sheet 9.

In the elastic component preparation step (3-1), the outer portions of the interposing sheets 9 in the leg elastic members 7d and 7e are cut so as to be separated from each other after the connection of the leg elastic members 7d and 7e. Thus, the elastic component 14 can be prepared.

Although the elastic component preparation step (3-1) of manufacturing the elastic component 14 using the individual interposing sheet 9 has been described, the elastic component preparation step (3-1) is not limited thereto. For example, it is preferable that continuous bodies and the leg elastic member 7 are connected in a state where the leg elastic members 7d and 7e are interposed between a continuous body in which the plurality of first interposing sheets 9 are continuous in the width direction W and a continuous body in which the plurality of second interposing sheets 9 are continuous in the width direction W, and the continuous bodies and the leg elastic member 7 are cut into individual elastic components 14. In this case, since the entire leg elastic members 7d and 7e can be interposed between the continuous bodies of the interposing sheet 9, the step of cutting the leg elastic members 7d and 7e disposed outside the interposing sheet 9 as described above can be omitted (when the continuous bodies is cut into individual elastic components 14, the leg elastic members 7d and 7e can also be cut by individual lengths).

Next, in the posture changing step (3-2), the posture of the elastic component 14 is changed so that the leg elastic members 7d and 7e have a predetermined posture with respect to the front elastic portion 2 and the rear elastic portion 3 in the elastic member connection step (3-5) described later, that is, the leg elastic members 7d and 7e have a posture extending in the extending direction. Specifically, in the present embodiment, the elastic component 14 is rotated by 90°.

In the sheet preparation step (3-3), one elastic sheet 6 having the front elastic portion 2 and the rear elastic portion 3 is prepared.

Although not illustrated, in the sheet weakening step, the elasticity in the weakening region WR including the weakening region WR1 and the weakening region WR2 of the elastic sheet 6 prepared in the sheet preparation step (3-3) is weakened.

In the cutting and separating step (3-4), the elastic sheet 6 is cut into the front elastic portion 2 and the rear elastic portion 3 along a cutting line C pre-set after the sheet weakening step. Further, in the cutting and separating step (3-4), the cut front elastic portion 2 and the cut rear elastic portion 3 are separated from each other in the extending direction.

In the elastic member connection step (3-5), the elastic component 14 disposed in the posture changing step (3-2) is connected to the front elastic portion 2 and the rear elastic portion 3 separated in the cutting and separating step (3-4). Specifically, in the elastic member connection step (3-5), the leg elastic members 7d and 7e are respectively disposed at elastic member connection positions pre-set on both sides of the region where the absorbent core 4d is to be disposed in the width direction W, and the elastic component 14 is connected to the front elastic portion 2 and the rear elastic portion 3 in a state where the leg elastic member 7 extends in the extending direction.

In the absorbent body connection step (3-6), the front part 4a of the absorbent body 4 is connected to the front connection region FC (hatched region in step (3-7)), and the rear part 4b of the absorbent body 4 is connected to the rear connection region RC (hatched region in step (3-7)). Specifically, the front part 4a and the rear part 4b of the absorbent body 4 are connected to the elastic component 14, the front elastic portion 2, and the rear elastic portion 3.

In the cutout forming step (3-7), the leg cutout 9a along the hole forming region pre-set in the elastic component 14 is formed.

According to the third embodiment, by connecting the absorbent body 4 to two elastic sheets (the front elastic portion 2 and the rear elastic portion 3) so as to extend across the two elastic sheets separated from each other, the elastic sheet in the portion covering the crotch part of the wearer can be omitted. Therefore, the usage amount of the elastic sheet 6 can be reduced.

Further, by connecting the leg elastic member 7 to the elastic sheet 6 (the front elastic portion 2 and the rear elastic portion 3) in a state of extending in the extending direction, elasticity in the extending direction can be imparted to the elastic sheet 6. Therefore, it is not necessary to extend the elastic sheet 6 in a plurality of directions, and handling of the elastic sheet 6 becomes easy.

In the third embodiment, it has been described that the steps (3-3) to (3-7) are performed using the individual elastic sheets 6, but it is preferable to perform the steps (3-3) to (3-7) in a state where the continuous body in which the plurality of elastic sheets 6 are continuous in the width direction W is conveyed in the width direction W.

Further, it is sufficient that the step (3-1) and the step (3-2) are performed before the execution of the step (3-5), and the execution times of the step (3-1) and the step (3-2) with respect to the step (3-3) and the step (3-4) are not limited.

Further, as in the first embodiment, the elastic member connection step (3-5) of the third embodiment can also be applied to a case where the elastic sheet 6 has a portion that covers the region of the crotch part of the wearer.

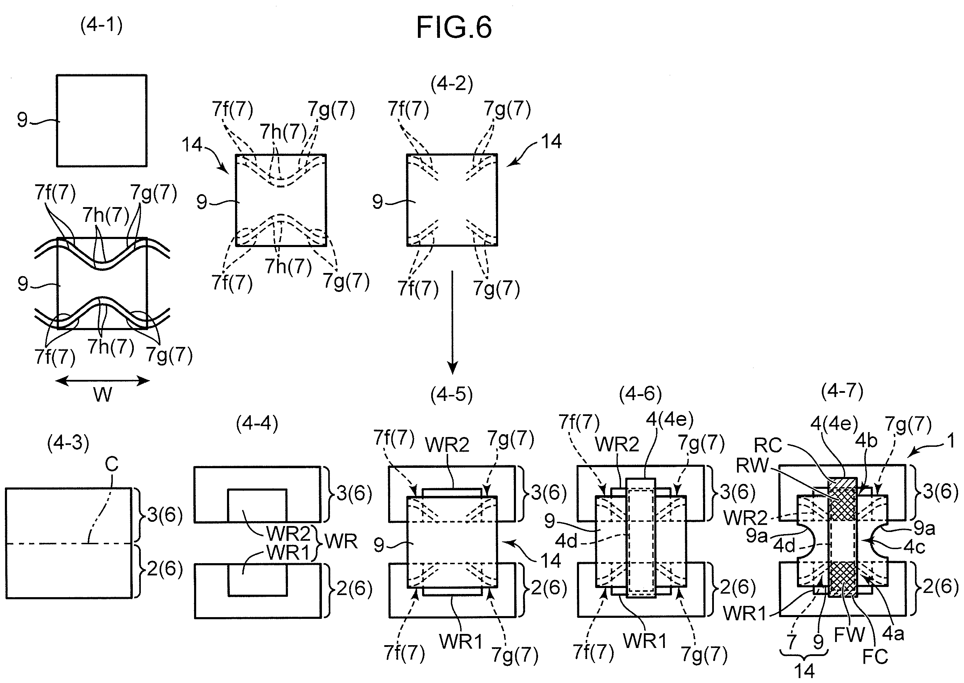

### <Fourth Embodiment (FIG. 6)>

In the third embodiment, after the elastic component 14 is prepared, the elastic member connection step (3-5) is performed by changing the posture of the elastic component 14 in the posture changing step (3-2), but a weakening step (4-2) for weakening the elasticity of a part of the leg elastic member 7 may be applied instead of the posture changing step (3-2).

Hereinafter, a method for manufacturing the wearable article 1 according to the fourth embodiment will be described. In the fourth embodiment, the same parts as those in the third embodiment are denoted by the same reference numerals, and the description thereof will be omitted.

Specifically, the method for manufacturing the wearable article 1 includes an elastic component preparation step (4-1), an elastic member weakening step (4-2), a sheet preparation step (4-3), a sheet weakening step (not illustrated), a cutting and separating step (4-4), an elastic member connection step (4-5), an absorbent body connection step (4-6), and a cutout forming step (4-7). In addition to the above steps, the method for manufacturing the wearable article 1 may include the folding-in-half step (1-5) in the first embodiment, and may include the side sealing step (1-6) or the step of attaching the surface tape T illustrated in FIG. 3. Through the above steps, the pants-type wearable article 1 (see FIG. 1) or the tape-type wearable article 1 (see FIG. 3) can be manufactured.

In the elastic component preparation step (4-1), first, the first interposing sheet (holding sheet) 9 is conveyed in the width direction W, and the leg elastic members 7 are supplied onto the first interposing sheet 9. Specifically, the leg elastic member 7 is supplied onto the first interposing sheet 9 so as to form a first portion 7f arranged on the first interposing sheet 9 in a state of being extended in the extending direction, a second portion 7g disposed on the first interposing sheet 9 in a state of being extended in the extending direction and being separated from the first portion 7f in the width direction W, and a connection portion 7h connecting the first portion 7f and the second portion 7g. Specifically, the leg elastic member is supplied along a path meandering in the conveyance direction (width direction W) of the first interposing sheet 9. The two leg elastic members 7 are supplied along a path parallel to each other to one side with respect to the central position in the extending direction of the first interposing sheet 9 as a boundary, and the two leg elastic members 7 are supplied along a path parallel to each other to the other side. The two leg elastic members 7 on one side and the two leg elastic members 7 on the other side are disposed so as to be closest to each other at the central position in the width direction W of the first interposing sheet 9 and away from each other as being away from the central position in the width direction W.

Further, in the elastic component preparation step (4-1), an adhesive is applied to the second interposing sheet 9, the second interposing sheet 9 is supplied to the first interposing sheet 9 so as to interpose the adhesive, and the interposing sheets 9 are pressed by nip rolls (not illustrated) in a state where the interposing sheets 9 are overlapped. As a result, the leg elastic members 7 are connected to the interposing sheets 9, and both the interposing sheets 9 are joined to each other. The connection is not limited to connection by an adhesive, and may be connection by welding using heat or ultrasonic waves. Similarly to the third embodiment, one of the two interposing sheets 9 can be omitted.

In the elastic component preparation step (4-1), the outer portion of the interposing sheet 9 in the leg elastic member 7 is cut so as to be separated from each other after the connection of the leg elastic member 7. Thus, the elastic component 14 can be prepared.

Although the elastic component preparation step (4-1) of manufacturing the elastic component 14 using the individual interposing sheet 9 has been described, the elastic component preparation step (4-1) is not limited thereto. For example, it is preferable that continuous bodies and the leg elastic member 7 are connected in a state where the leg elastic member 7 is interposed between a continuous body in which the plurality of first interposing sheets 9 are continuous in the width direction W and a continuous body in which the plurality of second interposing sheets 9 are continuous in the width direction W, and the continuous bodies and the leg elastic member are cut into individual elastic components 14. In this case, since the entire leg elastic member 7 can be interposed by the continuous bodies of the interposing sheet 9, the step of cuting the leg elastic member 7 disposed outside the interposing sheet 9 as described above can be omitted.

In the elastic member weakening step (4-2), the elasticity of a region pre-set as a region where the absorbent core 4d is to be disposed in the connection portion 7h of the leg elastic member 7 is weakened. Specifically, the elasticity can be weakened by finely cutting the corresponding portion of the connection portion 7h using a cutter having a plurality of blades arranged at a minute pitch in the width direction W. In addition, it is also possible to weaken the elasticity by intermittently adhering the connection portion 7h to the interposing sheet 9, and cutting the connection portion 7h between the adhering portions. The cutting of the connection portion 7h is not limited to cutting by a cutter, and may be cutting by heat or ultrasonic waves, for example.

Since the sheet preparation step (4-3), the sheet weakening step, and the cutting and separating step (4-4) are similar to the sheet preparation step (3-3), the sheet weakening step, and the cutting and separating step (3-4) in the third embodiment, the description thereof will be omitted.

In the elastic member connection step (4-5), the elastic component 14 is connected to the front elastic portion 2 and the rear elastic portion 3 in a state where the second portion 7g is disposed at the elastic member connection position set on the right side of the region where the absorbent core 4d is to be disposed and the first portion 7f is disposed at the elastic member connection position set on the left side of the region where the absorbent core 4d is to be disposed.

Since the absorbent body connection step (4-6) and the cutout forming step (4-7) are similar to the absorbent body connection step (3-6) and the cutout forming step (3-7) in the third embodiment, the description thereof will be omitted.

According to the fourth embodiment, in the elastic member weakening step (4-2), by weakening the elasticity of the region pre-set as the region where the absorbent core 4d is to be disposed in the connection portion 7h, it is possible to suppress the elasticity of the connection portion 7h of the leg elastic member 7 from being imparted to the absorbent core 4d after the absorbent body 4 is connected to the elastic sheet 6. As a result, it is possible to prevent the absorbent body 4 from shrinking in the orthogonal direction at the crotch part of the wearer when the wearable article 1 is worn, and thus, it is possible to prevent deterioration of the absorption performance of excreta.

In parallel with the steps (4-3) and (4-4) of applying various processing to the elastic sheet 6, the elastic component 14 in which the leg elastic members 7 are disposed in a predetermined positional relationship can be prepared. Therefore, unlike the case where the leg elastic members 7 are directly connected onto the elastic sheet 6, the productivity of the wearable article 1 can be improved.

In the fourth embodiment, it has been described that the steps (4-3) to (4-7) are performed using the individual elastic sheets 6, but it is preferable to perform the steps (4-3) to (4-7) in a state where the continuous body in which the plurality of elastic sheets 6 are continuous in the width direction W is conveyed in the width direction W.

In addition, the elastic component preparation step (4-1) only needs to be performed by the time of execution of the step (3-5), and the execution time of the elastic component preparation step (4-1) with respect to the step (4-3) and the step (4-4) is not limited.

Further, although the example in which the elastic member weakening step (4-2) is performed before the elastic member connection step (4-5) has been described, the elastic member weakening step (4-2) may be performed after the elastic member connection step (4-5).

The elastic member connection step (4-5) of the fourth embodiment can also be applied to a case where the elastic sheet 6 has a portion that covers the region of the crotch part of the wearer as in the first embodiment.

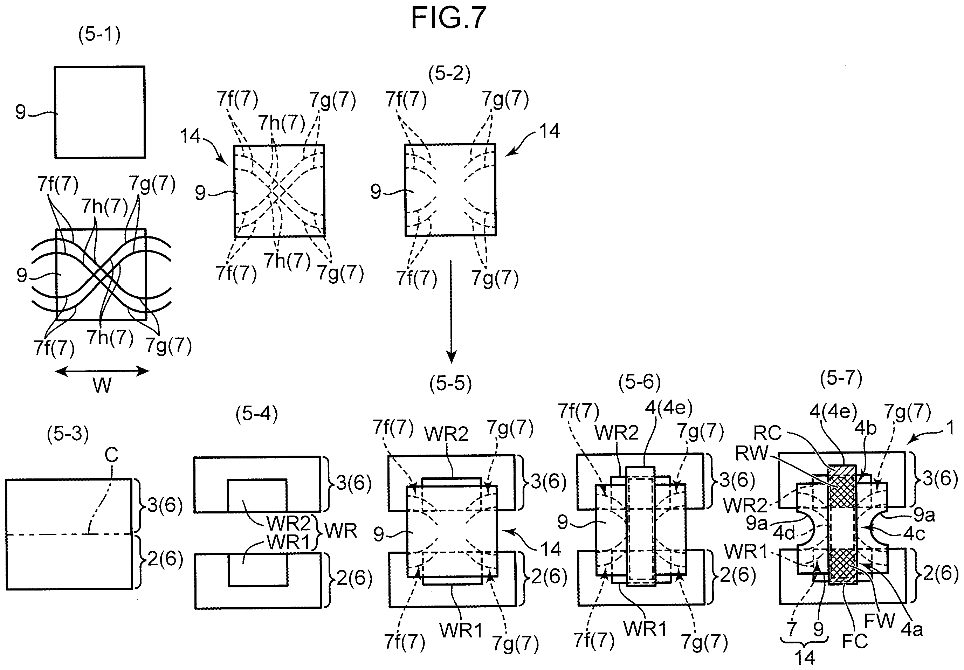

### <Fifth Embodiment (FIG. 7)>

In the fourth embodiment, an example has been described in which the path of the leg elastic member 7 disposed on one side in the extending direction and the path of the leg elastic member 7 disposed on the other side with respect to the central position in the extending direction of the first interposing sheet 9 as a boundary are separated in the extending direction. However, as in the fifth embodiment illustrated in FIG. 7, these paths may cross each other.

Specifically, the method for manufacturing the wearable article 1 includes an elastic component preparation step (5-1), an elastic member weakening step (5-2), a sheet preparation step (5-3), a sheet weakening step (not illustrated), a cutting and separating step (5-4), an elastic member connection step (5-5), an absorbent body connection step (5-6), and a cutout forming step (5-7). In addition to the above steps, the method for manufacturing the wearable article 1 may include the folding-in-half step (1-5) in the first embodiment, and may include the side sealing step (1-6) or the step of attaching the surface tape T illustrated in FIG. 3. Through the above steps, the pants-type wearable article 1 (see FIG. 1) or the tape-type wearable article 1 (see FIG. 3) can be manufactured.

The method for manufacturing the wearable article 1 of the fifth embodiment is different from the method for manufacturing the wearable article 1 of the fourth embodiment only in the elastic component preparation step (5-1). Therefore, a portion different from the elastic component preparation step (4-1) in the elastic component preparation step (5-1) will be described, and description of other steps will be omitted.

In the elastic component preparation step (5-1), unlike the elastic component preparation step (4-1), the leg elastic members 7 are supplied to the first interposing sheet 9 along paths that intersect each other at the central position in the width direction W of the first interposing sheet 9 and meander with respect to the width direction W. As a result, the leg elastic member 7 is supplied onto the first interposing sheet 9 so as to form the first portion 7f disposed on the first interposing sheet 9 in a state of being extended in the extending direction, the second portion 7g disposed on the first interposing sheet 9 in a state of being extended in the extending direction and being separated from the first portion 7f in the width direction W, and the connection portion 7h connecting the first portion 7a and the second portion 7g.

According to the fifth embodiment, the same effects as those of the fourth embodiment can be obtained.

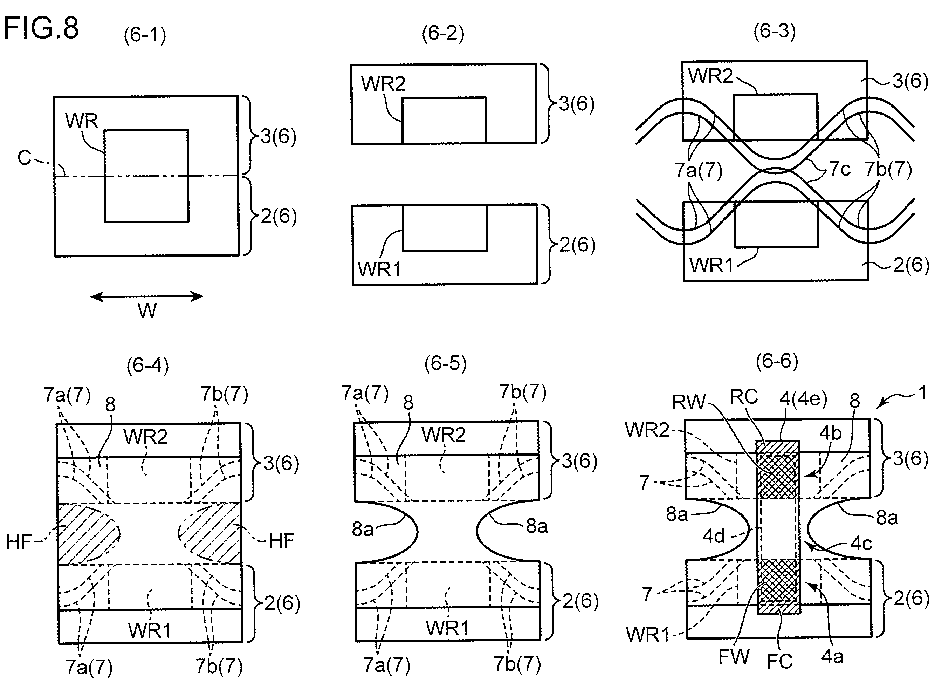

### <Sixth Embodiment (FIG. 8)>

In the sheet connection step (2-4) of the second embodiment, the covering sheet 8 is connected to the elastic sheet 6 so as to overlap only a part of the hole forming region for forming the leg hole, specifically, the front elastic portion 2 and the rear elastic portion 3. However, in the sheet connection step (6-4) of the sixth embodiment illustrated in FIG. 8, the covering sheet 8 is connected to the elastic sheet 6 so as to cover the entire hole forming region HF.

Hereinafter, a method for manufacturing the wearable article 1 according to the sixth embodiment will be described.

The method for manufacturing the wearable article 1 includes a sheet preparation step (not illustrated), a sheet weakening step (6-1), a cutting and separating step (6-2), an elastic member supply step (6-3), a sheet connection step (6-4), a cutout forming step (6-5), and an absorbent body connection step (6-6). In addition, the method for manufacturing the wearable article 1 may include the folding-in-half step (1-5) in the first embodiment in addition to the above steps, and may include the side sealing step (1-6) or the step of attaching the surface tape T illustrated in FIG. 3. Through the above steps, the pants-type wearable article 1 (see FIG. 1) or the tape-type wearable article 1 (see FIG. 3) can be manufactured.

The sheet preparation step, the sheet weakening step (6-1), the cutting and separating step (6-2), and the elastic member supply step (6-3) are the same as the sheet preparation step, the sheet weakening step (2-1), the cutting and separating step (2-2), and the elastic member supply step (2-3) in the second embodiment, respectively, and thus descriptions thereof will be omitted.

In the sheet connection step (6-4), unlike the sheet connection step (2-4) of the second embodiment, the covering sheet 8 is connected to the elastic sheet 6 so as to cover the entire hole forming region HF pre-set as a region for forming leg holes for passing the legs of the wearer, and to interpose the leg elastic member 7 with the elastic sheet 6. Specifically, in the sheet connection step (6-4), the covering sheet 8 is disposed to extend across the front elastic portion 2 and the rear elastic portion 3, the front part of the covering sheet 8 is connected to the front elastic portion 2, and the rear part of the covering sheet 8 is connected to the rear elastic portion 3.

In the cutout forming step (6-5), a cutout 8a for the leg hole along the hole forming region HF is formed after the sheet connection step (6-4). In the sixth embodiment, the hole forming region HF is set only between the front elastic portion 2 and the rear elastic portion 3, but the hole forming region HF may be set to overlap at least one of the front elastic portion 2 and the rear elastic portion 3. In this case, cutouts for leg holes are formed over the covering sheet 8, the front elastic portion 2, and the rear elastic portion 3.

In the absorbent body connection step (6-6), the front part 4a of the absorbent body 4 is connected to the front connection region FC (hatched portion), and the rear part 4b of the absorbent body 4 is connected to the rear connection region RC (hatched portion).

According to the sixth embodiment, since the covering sheet 8 can be disposed over the entire circumference of the edge of the leg hole of the wearable article 1, a uniform touch feeling can be provided to the wearer over the entire circumference of the leg hole. Thus, the wearing feeling can be improved.

Further, since the wearing feeling as described above can be improved using the covering sheet 8 covering the leg elastic members 7, the productivity of the wearable article 1 can be improved as compared with a case where a separate sheet is connected to the elastic sheet 6.

Note that, although the example in which the cutout forming step (6-5) is executed before the absorbent body connection step (6-6) has been described, these steps may be interchanged.

In addition, similarly to the second embodiment, it is preferable to execute steps (6-1) to (6-6) using a continuous body in which a plurality of elastic sheets 6 are continuous in the width direction W. In this case, in the sheet connection step (6-4), the continuous body of the plurality of covering sheets 8 in the width direction W can be connected to the elastic sheet 6 (the continuous body of the front elastic portion 2 and the rear elastic portion 3).

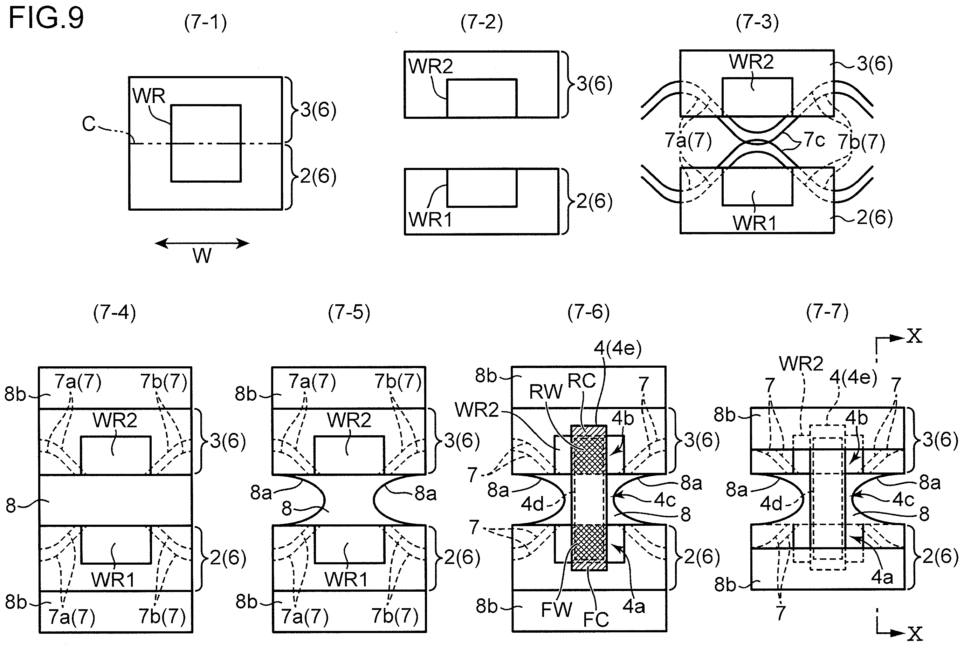

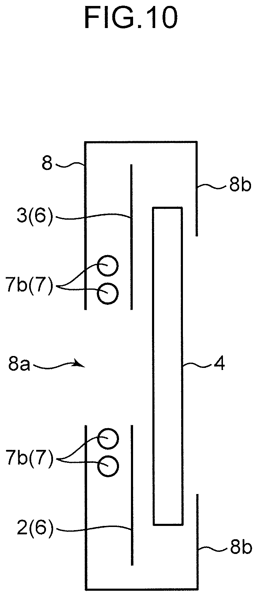

### <Seventh Embodiment (FIGS. 9 and 10)>

In the second embodiment and the sixth embodiment, the leg elastic member 7 and the covering sheet 8 are connected to the inner surface of the elastic sheet 6 facing the skin surface of the wearer. However, as in the seventh embodiment illustrated in FIGS. 9 and 10, the leg elastic member 7 and the covering sheet 8 can be connected to the outer surface of the elastic sheet 6 opposite to the skin surface of the wearer.

Further, in the sheet connection steps (2-4) and (6-4) of the second embodiment and the sixth embodiment, the covering sheet 8 is connected in a region between the waist side edges of the wearer in the extending direction of the elastic sheet 6. However, as in the sheet connection step (7-4) of the seventh embodiment, the covering sheet 8 may be connected to the elastic sheet 6 such that an extension part 8b extending in the direction away from the absorbent body 4 beyond the waist side edge of the wearer in the extending direction of the elastic sheet 6 is formed.

Hereinafter, a method for manufacturing the wearable article 1 according to the seventh embodiment will be described.

The method for manufacturing the wearable article 1 includes a sheet preparation step (not illustrated), a sheet weakening step (7-1), a cutting and separating step (7-2), an elastic member supply step (7-3), a sheet connection step (7-4), a cutout forming step (7-5), an absorbent body connection step (7-6), and an extension part connection step (7-7). In addition, the method for manufacturing the wearable article 1 may include the folding-in-half step (1-5) in the first embodiment in addition to the above steps, and may include the side sealing step (1-6) or the step of attaching the surface tape T illustrated in FIG. 3. Through the above steps, the pants-type wearable article 1 (see FIG. 1) or the tape-type wearable article 1 (see FIG. 3) can be manufactured.

The sheet preparation step, the sheet weakening step (7-1), and the cutting and separating step (7-2) are the same as the sheet preparation step, the sheet weakening step (2-1), the cutting and separating step (2-2), and the elastic member supply step (2-3) in the second embodiment, respectively, and thus the description thereof will be omitted.

In the elastic member supply step (7-3), as illustrated in FIGS. 9 and 10, unlike the elastic member supply step (2-3) of the second embodiment, the leg elastic member 7 is supplied to the surface of the elastic sheet 6 (the front elastic portion 2 and the rear elastic portion 3) on the side opposite to the skin surface.

In the sheet connection step (7-4), the covering sheet 8 is connected to the elastic sheet 6 so that the extension part 8b extending in a direction away from the absorbent body 4 beyond the waist side edge (in FIG. 9, the lower edge of the front elastic portion 2 and the upper edge of the rear elastic portion 3 are shown) of the wearer in the extending direction of the elastic sheet 6 (the front elastic portion 2 and the rear elastic portion 3) is formed. At this time, the covering sheet 8 is connected to the outer surface of the elastic sheet 6 opposite to the skin surface of the wearer so as to cover the leg elastic members 7.

Next, a cutout 8a for forming a leg hole is formed in the cutout forming step (7-5), and the absorbent body 4 is connected to the elastic sheet 6 and the covering sheet 8 in the absorbent body connection step (7-6).

In the extension part connection step (7-7), the extension part 8b is folded back so as to interpose the waist side edge of the elastic sheet 6 (the front elastic portion 2 and the rear elastic portion 3) and the end of the absorbent body 4, and the extension part 8b is connected to the elastic sheet 6 (the front elastic portion 2 and the rear elastic portion 3). In the extension part connection step (7-7), the extension part 8b may be connected to the absorbent body 4 in addition to the elastic sheet 6.

According to the seventh embodiment, since the covering sheet 8 can be interposed between the edge of the elastic sheet 6 and the end (end surface) of the absorbent body 4, and the skin surface of the wearer, the touch to the wearer can be improved. Thus, the wearing feeling can be improved.

Note that the order of the cutout forming step (7-5), the absorbent body connection step (7-6), and the extension part connection step (7-7) is not limited thereto on condition that the extension part connection step is executed after the absorbent body connection step.

In addition, similarly to the sixth embodiment, it is preferable to execute steps (7-1) to (7-7) using a continuous body in which a plurality of elastic sheets 6 are continuous in the width direction W. In this case, in the sheet connection step (7-4), the continuous body of the plurality of covering sheets 8 in the width direction W can be connected to the elastic sheet 6 (the continuous body of the front elastic portion 2 and the rear elastic portion 3).

Note that the steps in the first to seventh embodiments can be appropriately replaced with or combined with other embodiments.

In the sheet preparation step in the first to seventh embodiments, one elastic sheet 6 is prepared, and the sheet weakening step is executed on the elastic sheet 6, but the sheet preparation step is not limited thereto, and two elastic sheets 6 may be prepared. Specifically, in the sheet preparation step, one elastic sheet 6 having the front elastic portion 2 and one elastic sheet having the rear elastic portion 3 can be separately prepared. In this case, the sheet weakening step is simultaneously performed on the two elastic sheets 6.

Note that the above-described specific embodiments mainly include inventions having the following configurations.

In order to solve the above problems, the present invention provides a method for manufacturing a wearable article, the wearable article including: a front abdominal part disposed on an abdomen of a wearer; a back part disposed on buttocks of the wearer; and an absorbent body having a front part connected to the front abdominal part, a rear part connected to the back part, and an intermediate part extending from the front part to the rear part and disposed in a crotch part of the wearer. The method includes: a sheet preparation step of preparing at least one elastic sheet for forming the front abdominal part and the back part; a sheet weakening step of weakening elasticity of the elastic sheet in a front weakening region that is a front connection region to which a front part of the absorbent body is connected and that is pre-set as a region where an absorbent core of the absorbent body is disposed and a rear weakening region that is a rear connection region to which a rear part of the absorbent body is connected and that is pre-set as a region where the absorbent core is disposed in the at least one elastic sheet; and an absorbent body connection step of connecting a front part of the absorbent body to a front connection region of the at least one elastic sheet and connecting a rear part of the absorbent body to a rear connection region of the at least one elastic sheet. The sheet weakening step is simultaneously performed for the front weakening region and the rear weakening region.

According to the present invention, it is possible to simultaneously weaken the elasticity of the front connection region and the rear connection region in at least one elastic sheet for forming the front abdominal part and the back part. As a result, the efficiency of the weakening step can be improved as compared with the case of individually weakening the elasticity for the front connection region and the rear connection region. Therefore, the productivity of the wearable article can be improved.

In the sheet preparation step, a total of two elastic sheets including one elastic sheet for forming the front abdominal part and one elastic sheet for forming the back part can be prepared, and in the sheet weakening step, the elasticity of the two elastic sheets can be simultaneously weakened. However, in this case, it is necessary to adjust the relative positions of the two elastic sheets in order to simultaneously execute the sheet weakening step on the two elastic sheets.

Therefore, preferably in the method for manufacturing a wearable article, in the sheet preparation step, one elastic sheet having a front elastic portion for forming the front abdominal part and a rear elastic portion for forming the back part is prepared, and the sheet weakening step is performed on the one elastic sheet.

In this way, by performing the weakening step on one elastic sheet, the elasticity in the front connection region and the rear connection region can be simultaneously weakened. Therefore, the productivity of the wearable article can be improved as compared with the case of executing the weakening step while adjusting the relative positions of the two elastic sheets.

In the method for manufacturing a wearable article, the abdomen, the buttocks, and the crotch part of the wearer can be covered with one elastic sheet by forming a cutout for forming leg holes for passing the legs of the wearer with respect to one elastic sheet. However, since the absorbent body exists in the crotch part of the wearer, the elastic sheet can be omitted in a region covering the crotch part of the wearer.

Specifically, the method for manufacturing a wearable article preferably further includes: a cutting step of cutting the one elastic sheet into the front elastic portion and the rear elastic portion after the sheet weakening step; and a separating step of separating the cut front elastic portion and the cut rear elastic portion from each other in an extending direction of the absorbent body.

In this way, by connecting the absorbent body to two elastic sheets (the front elastic portion and the rear elastic portion) so as to extend across the two elastic sheets separated from each other, a region between the elastic sheets and divided by the absorbent body can be used as a leg hole. Therefore, the elastic sheet in the portion covering the crotch part of the wearer can be omitted, and the usage amount of the elastic sheet can be reduced.

The elasticity of the elastic sheet can also be used to impart elasticity in the extending direction of the absorbent body to the wearable article. However, in this case, it is necessary to manufacture a wearable article in a state where the elastic sheet is stretched in two directions, that is, a direction in which the absorbent body extends and a direction in which elasticity is originally required for the elastic sheet (a direction orthogonal to the direction in which the absorbent body extends), and handling of the elastic sheet becomes difficult.

Therefore, it is preferable that the method for manufacturing a wearable article further includes: an elastic member connection step of connecting the leg elastic member to the at least one elastic sheet in a state in which the leg elastic member is disposed at elastic member connection positions pre-set on both sides of a region in which the absorbent core is disposed in an orthogonal direction orthogonal to an extending direction of the absorbent body, and the leg elastic member is extended in the extending direction.

In this way, by connecting the leg elastic member to the elastic sheet in a state where the leg elastic member is extended in the extending direction of the absorbent body, the elasticity in the extending direction can be imparted to the elastic sheet. Therefore, it is not necessary to extend the elastic sheet in a plurality of directions in order to impart elasticity of the elastic sheet itself in the extending direction, and handling of the elastic sheet becomes easy.

The leg elastic member may be connected to the elastic sheet in an exposed state, but in this case, it is difficult to sufficiently secure a holding force for holding the leg elastic member with respect to the elastic sheet.

Therefore, it is preferable that the method for manufacturing a wearable article further includes: a sheet connection step of disposing a covering sheet on the at least one elastic sheet so as to cover the leg elastic member and connecting the covering sheet to the at least one elastic sheet.

In this way, by holding the leg elastic member between the covering sheet and the elastic sheet, it is possible to sufficiently secure a holding force for holding the leg elastic member with respect to the elastic sheet. Therefore, the leg elastic member can be reliably connected to the elastic sheet.

In the method for manufacturing a wearable article, in the sheet connection step, the covering sheet is connected to the at least one elastic sheet so as to cover an entire hole forming region pre-set as a region for forming a leg hole for passing a leg of a wearer, and it is preferable that the method for manufacturing a wearable article further includes a cutout forming step of forming a cutout along the hole forming region after the sheet connection step.

In this way, since the covering sheet can be disposed over the entire circumference of the edge of the leg hole of the wearable article, a uniform touch feeling can be provided to the wearer over the entire circumference of the leg hole. Thus, the wearing feeling can be improved.

Further, since the wearing feeling as described above can be improved using the covering sheet covering the leg elastic members, the productivity of the wearable article can be improved as compared with a case where a separate sheet is connected to the elastic sheet.

In the method for manufacturing a wearable article, in the sheet connection step, the covering sheet is connected to an outer surface of the at least one elastic sheet on a side opposite to a skin surface of the wearer such that an extension part extending in a direction away from the elastic sheet beyond a waist side edge of the wearer in the extending direction of the at least one elastic sheet is formed, and it is preferable that the method for manufacturing a wearable article further includes an extension part connection step of folding back the extension part so as to interpose an edge of the at least one elastic sheet and an end of the absorbent body and connecting the extension part to the at least one elastic sheet.

In this way, since the covering sheet can be interposed between the edge of the elastic sheet and the end (end surface) of the absorbent body, and the skin surface of the wearer, the touch to the wearer can be improved, whereby the wearing feeling can be improved.

Further, since the wearing feeling as described above can be improved using the covering sheet covering the leg elastic members, the productivity of the wearable article can be improved as compared with a case where a separate sheet is connected to the elastic sheet.

In the method for manufacturing a wearable article, in the elastic member connection step, the leg elastic member is connected to the at least one elastic sheet so as to form a first portion connected in a state of being extended in the extending direction to the elastic member connection position set on one side of the region in which the absorbent core is to be disposed, a second portion connected in a state of being extended in the extending direction to the elastic member connection position set on an other side of the region in which the absorbent core is to be disposed, and a connection portion connecting the first portion and the second portion, and it is preferable that the method for manufacturing a wearable article further comprises an elastic member weakening step of weakening elasticity of a region pre-set as a region where the absorbent core is to be disposed in the connection portion after the elastic member connection step.

For example, by supplying the leg elastic member along a path meandering in the orthogonal direction with respect to the elastic sheet and connecting the leg elastic member to the elastic sheet in an extended state, a part of the leg elastic member can be connected to the elastic sheet in a state of being arranged at the elastic member connection position. That is, by bonding the elastic sheet and the leg elastic member as described above, the leg elastic member can be connected to the elastic sheet in a state where the first portion is disposed on one side of the absorbent core and the second portion is disposed on the other side of the absorbent core.

However, in this case, the first portion and the second portion are connected by the connection portion, and elasticity by the connection portion is given to the absorbent core. Therefore, as described above, by weakening the elasticity of the region where the absorbent core is disposed in the connection portion, it is possible to prevent the elasticity of the connection portion from being imparted to the absorbent core. As a result, it is possible to prevent the absorbent body from shrinking in the orthogonal direction at the crotch part of the wearer when the wearable article is worn, and thus, it is possible to prevent deterioration of the absorption performance of excreta.

The method for manufacturing a wearable article preferably further includes: an elastic component preparation step of preparing an elastic component including a holding sheet and a pair of the leg elastic members connected to the holding sheet in a state of being extended in the orthogonal direction and in a state of being separated from each other in the extending direction; and a posture changing step of changing a posture of the elastic component so that the leg elastic member is in a posture extending in an extending direction. In the elastic member connection step, it is preferable that each leg elastic member is disposed at an elastic member connection position, and the elastic component is connected to the at least one elastic sheet in a state where the leg elastic member extends in an extending direction.

For example, the elastic component can be prepared by supplying the leg elastic member to the holding sheet along a path meandering in the orthogonal direction and separated from each other in the extending direction, and connecting the leg elastic member to the holding sheet in the extended state.

In this elastic component, a pair of leg elastic members is disposed with a gap. Therefore, by changing the posture of the elastic component in the posture changing step so that the gap overlaps the region where the absorbent core is to be disposed, each leg elastic member can be disposed at the elastic member connection position in the subsequent elastic member connection step.

In addition, it is possible to prepare an elastic component in which a pair of leg elastic members is disposed in a predetermined positional relationship in parallel with the step of performing various processing on the elastic sheet. Therefore, unlike the case where the leg elastic members are directly connected onto the elastic sheet, the productivity of the wearable article can be improved.

The method for manufacturing a wearable article preferably further includes: an elastic component preparation step of preparing an elastic component including a holding sheet, and the leg elastic member having a first portion connected to the holding sheet in a state of being extended in the extending direction, a second portion connected to the holding sheet in a state of being extended in the extending direction and being separated from the first portion in the orthogonal direction, and a connection portion connecting the first portion and the second portion; and an elastic member weakening step of weakening elasticity of a region pre-set as a region where the absorbent core is to be disposed in the connection portion. In the elastic member connection step, before or after the elastic member weakening step, it is preferable that the elastic component is connected to the at least one elastic sheet in a state where the first portion is disposed at the elastic member connection position set on one side of the region where the absorbent core is to be disposed and the second portion is disposed at the elastic member connection position set on an other side of the region where the absorbent core is to be disposed.

For example, by supplying the leg elastic member to the holding sheet along a path meandering in the orthogonal direction and connecting the leg elastic member to the holding sheet in an extended state, it is possible to prepare an elastic component in which the leg elastic member is disposed at a predetermined position. Specifically, in the elastic component, the first portion of the leg elastic member is connected to the holding sheet in an extended state in the extending direction. The second portion of the leg elastic member is connected to the holding sheet in an extended state in the extending direction and in a state of being separated from the first portion in the orthogonal direction. The first portion and the second portion are connected to each other by a connecting member of the leg elastic member.

However, when the first portion is disposed at the elastic member connection position set on one side of the region where the absorbent core is to be disposed, and the second portion is disposed at the elastic member connection position set on the other side of the region where the absorbent core is to be disposed, the connection portion overlaps the absorbent core, and the elasticity of the connection portion is given to the absorbent core.

Therefore, in the elastic member weakening step, by weakening the elasticity of the region pre-set as the region where the absorbent core is to be disposed in the connection portion, it is possible to suppress the elasticity of the connection portion of the leg elastic member from being imparted to the absorbent core after the absorbent body is connected to the elastic sheet. As a result, it is possible to prevent the absorbent body from shrinking in the orthogonal direction at the crotch part of the wearer when the wearable article is worn, and thus, it is possible to prevent deterioration of the absorption performance of excreta.

In addition, it is possible to prepare an elastic component in which the leg elastic members are disposed in a predetermined positional relationship in parallel with the step of performing various processing on the elastic sheet. Therefore, unlike the case where the leg elastic members are directly connected onto the elastic sheet, the productivity of the wearable article can be improved.

Further, the present invention provides a wearable article manufactured by the method for manufacturing a wearable article.

## Claims

1. A method for manufacturing a wearable article, the wearable article including: a front abdominal part disposed on an abdomen of a wearer; a back part disposed on buttocks of the wearer; and an absorbent body having a front part connected to the front abdominal part, a rear part connected to the back part, and an intermediate part extending from the front part to the rear part and disposed in a crotch part of the wearer, the method comprising:
a sheet preparation step of preparing at least one elastic sheet for forming the front abdominal part and the back part;
a sheet weakening step of weakening elasticity of the elastic sheet in a front weakening region that is a front connection region to which a front part of the absorbent body is connected and that is pre-set as a region where an absorbent core of the absorbent body is disposed and a rear weakening region that is a rear connection region to which a rear part of the absorbent body is connected and that is pre-set as a region where the absorbent core is disposed in the at least one elastic sheet; and
an absorbent body connection step of connecting a front part of the absorbent body to a front connection region of the at least one elastic sheet and connecting a rear part of the absorbent body to a rear connection region of the at least one elastic sheet, wherein
the sheet weakening step is simultaneously performed for the front weakening region and the rear weakening region.

2. The method for manufacturing a wearable article according to claim 1, wherein
in the sheet preparation step, one elastic sheet having a front elastic portion for forming the front abdominal part and a rear elastic portion for forming the back part is prepared, and
the sheet weakening step is performed on the one elastic sheet.

3. The method for manufacturing a wearable article according to claim 2, further comprising:
a cutting step of cutting the one elastic sheet into the front elastic portion and the rear elastic portion after the sheet weakening step; and
a separating step of separating the cut front elastic portion and the cut rear elastic portion from each other in an extending direction of the absorbent body.

4. The method for manufacturing a wearable article according to any one of claims 1 to 3, further comprising:
an elastic member connection step of connecting the leg elastic member to the at least one elastic sheet in a state in which the leg elastic member is disposed at elastic member connection positions pre-set on both sides of a region in which the absorbent core is disposed in an orthogonal direction orthogonal to an extending direction of the absorbent body, and the leg elastic member is extended in the extending direction.

5. The method for manufacturing a wearable article according to claim 4, further comprising:
a sheet connection step of disposing a covering sheet on the at least one elastic sheet so as to cover the leg elastic member and connecting the covering sheet to the at least one elastic sheet.

6. The method for manufacturing a wearable article according to claim 5, wherein in the sheet connection step, the covering sheet is connected to the at least one elastic sheet so as to cover an entire hole forming region pre-set as a region for forming a leg hole for passing a leg of a wearer, and
the method for manufacturing a wearable article further comprises a cutout forming step of forming a cutout along the hole forming region after the sheet connection step.

7. The method for manufacturing a wearable article according to claim 5 or 6, wherein in the sheet connection step, the covering sheet is connected to an outer surface of the at least one elastic sheet on a side opposite to a skin surface of the wearer such that an extension part extending in a direction away from the elastic sheet beyond a waist side edge of the wearer in the extending direction of the at least one elastic sheet is formed, and
the method for manufacturing a wearable article further comprises an extension part connection step of folding back the extension part so as to interpose an edge of the at least one elastic sheet and an end of the absorbent body and connecting the extension part to the at least one elastic sheet.

8. The method for manufacturing a wearable article according to any one of claims 4 to 7, wherein in the elastic member connection step, the leg elastic member is connected to the at least one elastic sheet so as to form a first portion connected in a state of being extended in the extending direction to the elastic member connection position set on one side of the region in which the absorbent core is to be disposed, a second portion connected in a state of being extended in the extending direction to the elastic member connection position set on an other side of the region in which the absorbent core is to be disposed, and a connection portion connecting the first portion and the second portion, and
the method for manufacturing a wearable article further comprises an elastic member weakening step of weakening elasticity of a region pre-set as a region where the absorbent core is to be disposed in the connection portion after the elastic member connection step.

9. The method for manufacturing a wearable article according to claim 4, the method further comprising:
an elastic component preparation step of preparing an elastic component including a holding sheet and a pair of the leg elastic members connected to the holding sheet in a state of being extended in the orthogonal direction and in a state of being separated from each other in the extending direction; and
a posture changing step of changing a posture of the elastic component so that the leg elastic member is in a posture extending in an extending direction, wherein
in the elastic member connection step, each leg elastic member is disposed at an elastic member connection position, and the elastic component is connected to the at least one elastic sheet in a state where the leg elastic member extends in an extending direction.

10. The method for manufacturing a wearable article according to claim 4, the method further comprising:
an elastic component preparation step of preparing an elastic component including a holding sheet, and the leg elastic member having a first portion connected to the holding sheet in a state of being extended in the extending direction, a second portion connected to the holding sheet in a state of being extended in the extending direction and being separated from the first portion in the orthogonal direction, and a connection portion connecting the first portion and the second portion; and
an elastic member weakening step of weakening elasticity of a region pre-set as a region where the absorbent core is to be disposed in the connection portion, wherein
in the elastic member connection step, before or after the elastic member weakening step, the elastic component is connected to the at least one elastic sheet in a state where the first portion is disposed at the elastic member connection position set on one side of the region where the absorbent core is to be disposed and the second portion is disposed at the elastic member connection position set on an other side of the region where the absorbent core is to be disposed.

11. A wearable article manufactured by the method for manufacturing a wearable article according to any one of claims 1 to 10.
